# EUROPEAN PATENT SPECIFICATION

(11) **EP 1 886 711 B1**
(45) Date of publication and mention of the grant of the patent: **04.04.2018**
(21) Application number: 06756918.6
(22) Date of filing: 02.06.2006
(51) Int. Cl.: A61M 25/10, A61M 39/00, A61B 17/12

(54) **BALLOON CATHETER**
BALLONKATHETER
CATHETER A BALLONNET

(30) Priority: 02.06.2005 JP 2005162348
(43) Date of publication of application: 13.02.2008
(60) Divisional application: 11007963.9
(73) Proprietor: Olympus Corporation, Tokyo 192-8507 (JP)
(72) Inventor: NAGAMATSU, Ryuji, Tokyo 1920919 (JP); YANUMA, Yutaka, Tokyo 1860004 (JP)
(74) Representative: von Hellfeld, Axel
(86) International application number: PCT/JP2006/311083
(87) International publication number: WO 2006/129787

(56) References cited:
- JP-A- 04 309 370
- JP-A- 05 038 367
- JP-A- 06 154 320
- JP-A- 2004 329 720
- US-A- 3 799 171
- US-A- 4 116 201
- US-A- 4 205 683
- US-A- 4 654 027
- US-A- 4 793 351
- US-A- 5 453 097
- US-A- 5 749 853

## Description

### BACKGROUND OF THE INVENTION

### Field of the Invention

The present invention relates to a balloon catheter that is used by being introduced into a body cavity.

### Description of Related Art

As a method of extracting stones in a common bile duct to the duodenum endoscopically, a method of arranging a balloon in a bile duct while performing X-ray imaging and then expanding the balloon at a high pressure to expand the bile duct opening to an extent that allows stones to be extracted has presently become widely prevalent. A balloon catheter as shown in Patent Document 1 (Japanese Unexamined Patent Application, First Publication No. 2002-143311) is, for example, used for this method.

A syringe into which a fluid is inserted for expanding the balloon via an open-close valve is attached to the balloon catheter. For that reason, it is possible to expand the balloon by supplying the fluid from the syringe with the open-close valve opened, and after expanding the balloon, it is possible to maintain the expanded state of the balloon by preventing backflow of the fluid from the balloon by closing the open-close valve. Also, when contracting the balloon, it is possible to do so by opening the open-close valve to discharge the fluid from the balloon to the syringe.

In the above-mentioned conventional balloon catheter, when the operator performs the endoscope operation and balloon expansion operation, it is possible to proceed without assistance until the syringe operation. However, in order to maintain the expansion of the balloon, the procedure of operating the open-close valve while pressing down the syringe cannot be performed alone, and so a coordinated operation with an assistant is required. For that reason, the expansion-and-contraction operation of the balloon cannot be performed up to the expectations of the operator. Accordingly, the procedure time increases, which leads to an increase in X-ray exposure.

The present invention was achieved in view of the above circumstances, and has as its object to provide a balloon catheter enabling an operator to perform expansion or contraction of the balloon without assistance and enabling a shortening of the procedure time by improving operability.

Document US 5,749,853 discloses a remote controller. The remote controller is connected electrically to an instrument via an open contact surface and controls a fluid supplied from a syringe to a balloon by an increase-pressure switch, a decrease-pressure switch and a rapid-decrease-pressure being operated. The remote controller cannot pass a fluid supplied from the syringe to the balloon and, simultaneous with the supply from the syringe being stopped, automatically prevent backflow of the fluid that is supplied to the balloon to the syringe.

Document US 4,654,027 discloses a valve seat. The valve only passes a fluid to a fluid supply source side. The valve cannot automatically (i.e. in all cases) pass a fluid supplied from the fluid supply source side.

Document US 3,799,171 discloses a device having a configuration in which a fluid supplied from a fluid supply source passes into the compressible plug by depressing a proximal end side of a compressible plug and, simultaneous with the supply from the fluid supply source being stopped, prevents backflow of the fluid that is supplied to the balloon to the fluid supply source side by releasing a pressing force to the proximal end side of a compressible plug. The device cannot automatically pass a fluid supplied from a fluid supply source into the compressible plug because it is necessary to depress the proximal end side of the compressible plug by pushing a syringe manually. In a similar way, the device cannot automatically prevent backflow of the fluid that is supplied to the balloon to the fluid supply source side because it is necessary to release depression to the proximal end side of the compressible plug by pulling back the syringe manually.

Document US 4,793,351 discloses devices having valves which have to be operated manually, i.e. do not operate automatically.

US 4,116,201 discloses a further balloon catheter device.

### SUMMARY OF THE INVENTION

The present invention adopts the following constitutions in order to solve the aforesaid problems.

A balloon catheter according to the present invention is provided in accordance with claim 1.

This balloon catheter supplies a fluid from a fluid supply source to a balloon to expand the balloon and, simultaneous with stopping the fluid supply, can maintain the expansion of the balloon by confining the fluid in the balloon. Also, it is possible to contract the balloon by releasing the fluid to outside of the balloon by the fluid releasing portion.

Also, in the case of independently arranging a supply operating member, a fluid regulating portion, and a fluid operating portion from the fluid supply source within the range that the operator can control with one hand, when the operator performs with one hand the fluid supply operation from the fluid supply source to the balloon, it is possible to perform expansion of the balloon and maintenance of the expansion with one hand. In addition, it is possible to contract the balloon by only a fluid releasing operation with one hand by the fluid releasing portion.

The balloon catheter according to the present invention, wherein it is preferable to further provide an operating portion that has the fluid regulating portion and the fluid releasing portion; with a fixing portion that detachably mounts the operating portion on an endoscope operating portion of an endoscope or vicinity thereof is provided at the operating portion.

With this balloon catheter, the operator controlling the endoscope can perform not only the expansion and maintenance operations of a balloon but also the balloon contraction operation in a stable state in the case of performing the fluid supply operation from the fluid supply source.

The balloon catheter according to the present operation wherein the fluid releasing portion is preferably disposed more to the balloon side than the fluid regulating portion.

With this balloon catheter, when releasing the fluid from the balloon to the outside, it is possible to discharge the fluid from the fluid releasing portion to the outside before the fluid reaches the fluid releasing portion. Accordingly, when the operator performs fluid supply alone, it is possible to smoothly perform contraction of the balloon alone.

The balloon catheter according to the present invention, preferably further provided with a valve portion in which is disposed a connection opening that is connected with the base end side of the catheter shaft, an attachment opening that is connected with the fluid supply source that supplies fluid for expanding the balloon, and a release opening to the atmosphere; wherein the fluid releasing portion is disposed in the valve portion as a plug portion that selects either one of a first pipeline that allows communication between the connection opening and the attachment opening *and a second pipeline that branches from the first pipeline and allows communication between the connection opening and the release opening; and the fluid regulating portion is disposed in the first pipeline between the position where the plug portion is disposed and the attachment opening.

With this balloon catheter, when supplying fluid from the fluid supply source in the state of the fluid supply source being connected to the attachment opening of the valve portion, the catheter shaft being connected to the connection opening, and the first pipeline allowing communication between the connection opening and the attachment opening, it is possible to expand the balloon by supplying the fluid to the lumen of the catheter shaft.

At this time, since the check valve is disposed in the valve portion, when maintaining the expansion of the balloon, even without particularly performing the operation of the fluid supply source and the balloon catheter, it is possible to favorably prevent backflow of the supplied fluid to the fluid supply source side, and possible to maintain the expanded state of the balloon, and possible to perform expansion of the balloon and maintenance thereof with the operation of one hand.

Also, since the check valve is disposed between the attachment opening and the plug portion, when contracting the balloon, by switching the plug portion to allow communication between the connection opening and the release opening, it is possible to discharge the fluid from the discharge opening to the outside with an operation by one hand only. Therefore, it is possible to perform the series of operation of expanding a balloon, maintenance thereof, and contraction with one hand and therefore possible for an operator to operate the balloon catheter alone. According to the present invention, it is possible for an operator to perform the operations of expanding a balloon, maintenance thereof, and contraction alone without requiring the help of an assistant, and possible to shorten the procedure time by improving the balloon operability.

### BRIEF DESCRIPTION OF THE DRAWINGS

FIG 1 is a view showing the overall outline of the balloon catheter according to the first embodiment of the present invention.
FIG. 2A is a plan view showing the state when expanding the balloon of the valve portion of the balloon catheter according to the first embodiment of the present invention and when maintaining the expansion.
FIG. 2B is a plan view showing the state when contracting the balloon of the valve portion of the balloon catheter according to the first embodiment of the present invention.
FIG. 3A is a left elevation view showing the state of the check valve that is disposed in the valve portion of the balloon catheter according to the first embodiment of the present invention stopping the flow of air.
FIG. 3B is a side cross-sectional view showing the state of the check valve that is disposed in the valve portion of the balloon catheter according to the first embodiment of the present invention stopping the flow of air.
FIG. 3C is a right elevation view showing the state of the check valve that is disposed in the valve portion of the balloon catheter according to the first embodiment of the present invention stopping the flow of air.
FIG. 3D is a side cross-sectional view showing the state of the check valve that is disposed in the valve portion of the balloon catheter according to the first embodiment of the present invention passing air.

### DETAILED DESCRIPTION OF THE INVENTION

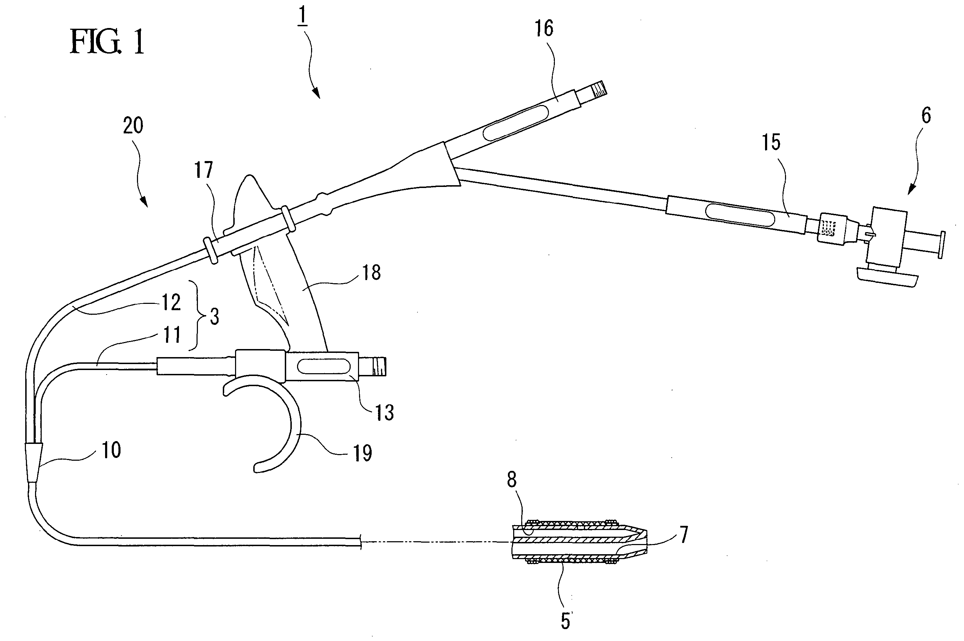

The first embodiment according to the present invention shall be described with reference to FIG. 1 to FIG. 3D.

A balloon catheter 1 according to the present embodiment is as shown in FIG. 1 able to advance and retreat in a channel of an endoscope not illustrated along a guide wire not illustrated. This balloon catheter 1 is provided with a catheter shaft 3 that is narrow and flexible, a balloon 5 that is disposed on the distal end side of the catheter shaft 3, and a valve portion 6 that is connected with the base end side of the catheter shaft 3.

The catheter shaft 3 is formed from a guide wire lumen 7 that is flexible and through which a guide wire is passed, a balloon lumen (lumen) 8 through that is made continuous with the balloon 5 and through which air (fluid) is passed for expanding and contracting the balloon 5, and a contrast agent lumen not illustrated through which a contrast agent is passed.

The catheter shaft 3 moreover is branched by a branching portion 10 disposed on the base end side into a first shaft 11 in which the guide wire lumen 7 is arranged and a second shaft 12 in which the balloon lumen 8 and the contrast agent lumen are arranged.

A wire mouthpiece 13 for passing the guide wire is disposed at the base end of the first shaft 11.

The base end side of the second shaft 12 further branches into two, with a valve mouthpiece 15 that is connected to the valve portion 6, and a contrast agent mouthpiece 16 for flowing in the contrast agent being disposed.

On the wire mouthpiece 13 is disposed a support portion 18 that can detachably support a connector 17 that is arranged on the second shaft 12 for positioning the second shaft 12 with respect to the first shaft 11 at a predetermined angle and spacing.

A fixing portion 19 that is formed in a substantial C-shape to be engageable with the operation portion not illustrated on the endoscope or thereabouts is disposed on the support portion 18 for positioning the wire mouthpiece 13 with respect to the endoscope or the like.

Note that the balloon catheter 1 is provided with a total operating portion (operating portion) 20 that is constituted by the members from the branching portion 10 to the valve portion 6 on the proximal side thereof and the contrast agent mouthpiece 16.

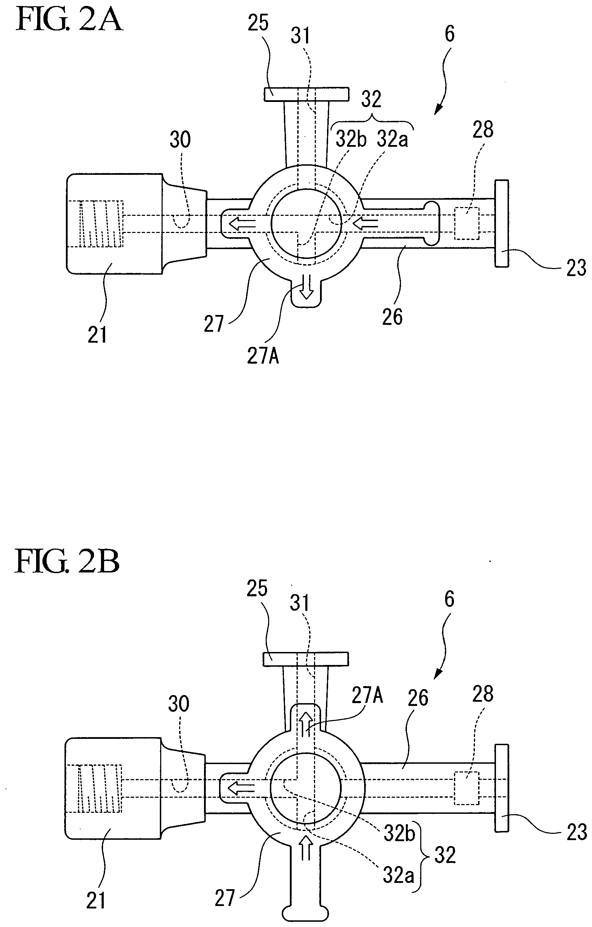

The valve portion 6 is, as shown in FIG. 2A and FIG. 2B, provided with a valve body 26 in which is disposed a connection opening 21 that is connected to a valve mouthpiece 15 of the second shaft 12, an attachment opening 23 that is connected to a syringe (fluid supply source) not illustrated that supplies air for expanding the balloon 5, and a release opening 25 to the atmosphere; a plug portion (fluid releasing portion) 27 that selects either one of a first pipeline 30 and a second pipeline 31 and thus makes continuous the connection opening 21 and the release opening 25, or the attachment opening 23 and the release opening 25; and a check valve (fluid regulating portion) 28 that is disposed in the first pipeline 30 between the plug portion 27 and the attachment opening 23 and allows the passage of fluid heading from the attachment opening 23 side to the connection opening 21 side while blocking fluid heading from the connection opening 21 side or opening 25 side to the attachment opening 23 side.

At the valve body 26 is disposed the first pipeline 30 that allows communication between the connection opening 21 and the attachment opening 23 and the second pipeline 31 that branches from the first pipe 30 and allows communication between the connection opening 21 and the release opening 25. In the plug portion 27 is disposed a T-pipeline 32 that consists of a main pipe 32a and a support pipe 32b that is connected to the middle of the main pipe 32a to communicate therewith and constitute a T-tube. On the surface of the plug portion 27 is provided an indicator portion 27A for alignment of the first pipeline 30 and the second pipeline 31 with both end positions of the main pipe 32a and the end portion position of the support pipe 32b.

When the plug portion 27 is arranged in the position shown in FIG. 2A, the T-pipeline 32 puts the connection opening 21 and the attachment opening 23 in a state of communication since the first pipeline 30 and the main pipe 32a are in communication. On the other hand, since the end portion of the support pipe 32b and the second pipeline 31 are facing different directions, the T-pipeline 32 blocks the opening 21 and the release opening 25. Also, when the plug portion 27 is arranged at the position shown in FIG. 2B, the end portion of the support pipe 32b faces the connection opening 21 side of the first pipeline 30, and one end of the main pipe 32a faces the release opening 25 side of the second pipeline 31. Thereby, while the connection opening 21 and the release opening 25 are put in a state of communication, the other end of the main pipe 32a is blocked off so that the connection opening 21 and the attachment opening 23 are blocked.

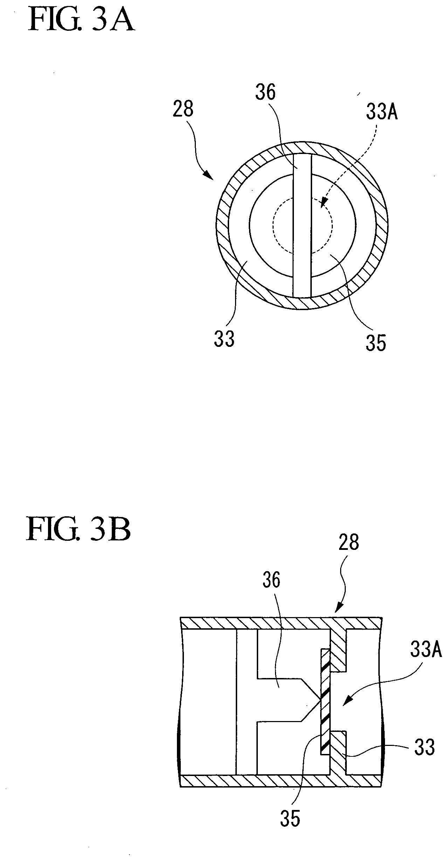

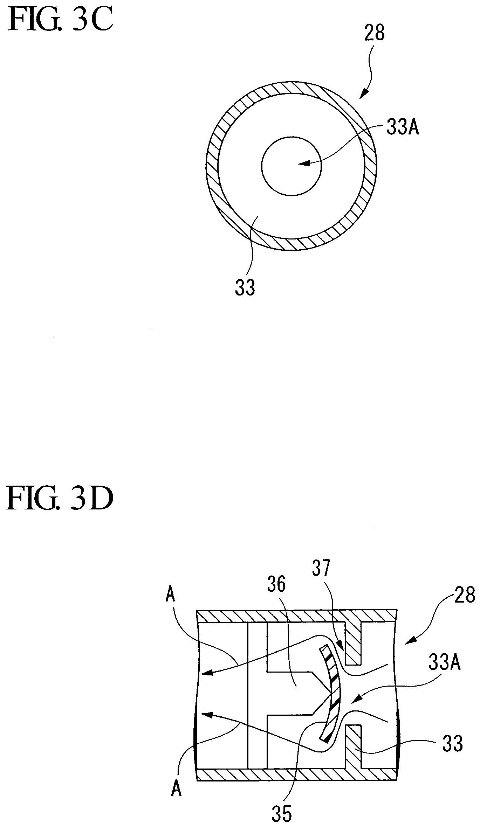

The check valve 28 is as shown in FIG. 3A, FIG. 3B, and FIG. 3C provided with an orifice portion 33 that is disposed projecting inward in the diameter direction of the first pipeline 30 and with an orifice hole 33A formed in the center thereof, a lid portion 35 that is constituted with a flexible material such as silicon and has a larger diameter than the orifice hole 33A so that the edge portion is in contact with the orifice portion 33, and a pressing portion 36 that presses at a predetermined pressure from a predetermined position the center portion of the lid portion 35 toward the attachment opening 23 side.

In this check valve 28, as shown in FIG 3B, the orifice hole 33A is plugged as a result of the lid portion 35 being pressed on the orifice portion 33 by air when air is flowing from the connection opening 21 side to the attachment opening 23 side. On the other hand, when air flows from the attachment opening 23 side to the connection opening 21 side, as shown in FIG. 3D, the lid portion 35 moves so as to separate from the orifice portion 33. However, because the movement of the lid portion 35 is restricted by the pressing portion 36, the lid portion 35 curves. At this time, a gap 37 is formed between the edge portion of the lid portion 35 and the orifice portion 33. Accordingly, air flows via the gap 37 as shown by the arrows A in the drawing.

Next, the exemplary method of operating the balloon catheter 1 according to the present embodiment and the operation/effect shall be described.

First, an endoscope not illustrated is inserted into a body cavity, and a guide wire not illustrated is inserted until a predetermined position in a bile duct via the endoscope by a widely known method and operation, and a predetermined treatment is completed by a predetermined treatment tool.

Next, the balloon catheter 1 is prepared by connecting the connection opening 21 of the valve portion 6 to the valve mouthpiece 15 that is disposed on the second shaft 12 and connecting the syringe 52 to the attachment opening 23 of the valve portion 6.

Then, the distal end side of the catheter shaft 3 is inserted into the bile duct via the endoscope while inserting the guide wire into the guide wire lumen 7 of the balloon catheter 1, and the fixing portion 19 of the balloon catheter 1 is fixed to a predetermined position.

Also, by flowing the contrast agent into the contrast agent lumen not illustrated from the contrast agent mouthpiece 16, the treatment proceeds while performing X-ray imaging.

In the case of expanding the balloon 5, the operator, while grasping the endoscope with one hand throughout, turns the plug portion 27 of the valve portion 6 with the other hand to adjust the direction of the T-pipeline 32 so as to put the connection opening 21 and the attachment opening 23 in a state of communication. Thereby, the syringe 52 and the balloon lumen 8 communicate.

In this state, the syringe 52 is operated with the other hand to flow a predetermined quantity of air into the valve portion 6. At this time, due to the air pressure, the lid portion 35 of the check valve 28 tries to move in the direction to separate from the orifice portion 33. However, because the center portion abuts the pressing portion 36, the edge portion side curves, and the gap 37 is formed between the edge portion of the lid portion 35 and the orifice portion 33. The air flows then through the first pipeline 30 via this gap 37. Thereby, the air flows into the balloon 5, causing it to expand.

When the predetermined quantity of air has flowed, the pressure on the side of the balloon 5 rises instead of the air pressure from the syringe 52 decreasing. Due to this air pressure, the lid portion 35 is pressed onto the orifice portion 33, and so the orifice hole 33A is plugged. For this reason, although the air attempts to flow back to the syringe 52 side it is blocked and so the pressure in the balloon 5 is maintained so that its expansion state is maintained.

In this state, stones and the like are cleared away from within the bile duct and discharged therefrom.

When contracting the balloon 5, the operator, while grasping the endoscope with one hand throughout as described above, turns the plug portion 27 of the valve portion 6 with the other hand to adjust the direction of the T-pipeline 32 so as to put the connection opening 21 and the release opening 25 in a state of communication as shown in FIG. 2B. Thereby, the air in the balloon 5 and the balloon lumen 8 is discharged to the atmosphere from the release opening 25. Accompanying this, the balloon 5 contracts.

Thus, by removing the balloon catheter 1, or the entire endoscope, from the body cavity, the treatment by the balloon 5 is completed.

According to the balloon catheter 1, since the check valve 28 is disposed in the valve portion 6, when maintaining the expansion of the balloon 5, it is possible to suitably prevent the supplied air from flowing back to the syringe 52 side and maintain the expanded state of the balloon 5 without particularly performing operation of the syringe 52 or the balloon catheter 1.

Also, since the check valve 28 is arranged in the first pipeline 30 between the attachment opening 23 and the plug portion 27, when contracting the balloon 5, it is possible to discharge the air from the release opening 25 to the outside via the second pipeline 31 just by the operation of bringing the connection opening 21 and the release opening 25 into communication by switching the plug portion 27. Accordingly, it is possible for an operator to perform the operations of expanding a balloon, maintenance thereof, and contraction with one hand, and so possible to perform work without requiring the help of an assistant.

For that reason, it is possible to ease the stress on the operator and cut down on X-ray exposure by shortening the procedure time, and also possible to realize a reduction in labor and other costs.

### INDUSTRIAL APPLICABILITY

The present invention can be used for a device that is introduced to a body cavity of a living body, and a system that includes such a device.

## Claims

1. A balloon catheter (1) comprising:
a catheter shaft (3) having flexibility with a lumen formed inside;
a balloon (5) that is disposed at the distal end side of the catheter shaft (3) and in communication with the lumen;
a fluid regulating portion (28) that passes a fluid that is supplied from a fluid supply source to the balloon and, simultaneous with the supply from the fluid supply source being stopped, prevents backflow of the fluid that is supplied to the balloon (5) to the fluid supply source side;
a fluid releasing portion (27) that releases the fluid that is supplied to the balloon (5) to outside of the balloon,
a valve portion (6) that includes a connection opening (21) connected to the distal end side of the catheter shaft (3), an attachment opening (23) connected to the fluid supply source which supplies the fluid for expanding the balloon (5), and a release opening (25) connected to an atmosphere,
**characterized in that**
the fluid releasing portion (27) is disposed in the valve portion (6) as a plug portion which selects either a first pipeline (30) which communicates the connection opening (21) with the attachment opening (23) and a second pipeline (31) which branches from the first pipeline (30) and communicates the connection opening (21) with the release opening (25), and
the fluid regulating portion (28) is disposed in a position between a position where the fluid releasing portion (27) is disposed and the attachment opening (23) at the first pipeline (30).

2. The balloon catheter according to claim 1,
further provided with an operating portion that has the fluid regulating portion (28) and the fluid releasing portion (27);
wherein a fixing portion (21) that detachably mounts the operating portion on an endoscope operating portion (15) of an endoscope or vicinity thereof is provided at the operating portion.

3. The balloon catheter according to claim 1,
wherein the fluid releasing portion (27) is disposed more to the balloon side than the fluid regulating portion (28).

## Patentansprüche

1. Ballonkatheter (1), umfassend:
einen Katheterschaft (3), der Flexibilität durch ein im Inneren gebildetes Lumen aufweist;
einen Ballon (5), der an der Distalendseite des Katheterschafts (3) angeordnet ist und mit dem Lumen in Verbindung steht;
einen Fluidregulierungsabschnitt (28), der ein Fluid weitergibt, das von einer Fluidversorgungsquelle an den Ballon geliefert wird, und gleichzeitig mit dem Stillstand der Versorgung der Fluidversorgungsquelle den Rückfluss des an den Ballon (5) gelieferten Fluids an die Fluidversorgungsquellenseite verhindert;
einen Fluidfreigabeabschnitt (27), der das Fluid, das an den Ballon (5) geliefert wird, an die Außenseite des Ballons freigibt,
einen Ventilabschnitt (6), der eine Verbindungsöffnung (21), die mit der Distalendseite des Katheterschafts (3) verbunden ist, eine Befestigungsöffnung (23), die mit der Fluidversorgungsquelle verbunden ist, die das Fluid zum Ausdehnen des Ballons (5) liefert, und eine Freigabeöffnung (25), die mit einer Atmosphäre verbunden ist, umfasst,
**dadurch gekennzeichnet, dass**
der Fluidfreigabeabschnitt (27) in dem Ventilabschnitt (6) als ein Anschlussabschnitt angeordnet ist, der entweder eine erste Leitung (30), die die Verbindungsöffnung (21) mit der Befestigungsöffnung (23) verbindet, oder eine zweite Leitung (31), die von der ersten Leitung (30) abzweigt und die Verbindungsöffnung (21) mit der Freigabeöffnung (25) verbindet, auswählt, und
der Fluidregulierungsabschnitt (28) in einer Position zwischen einer Position, in der der Fluidfreigabeabschnitt (27) angeordnet ist, und der Befestigungsöffnung (23) an der ersten Leitung angeordnet ist.

2. Ballonkatheter nach Anspruch 1,
der ferner mit einem Bedienabschnitt versehen ist, der den Fluidregulierungsabschnitt (28) und den Fluidfreigabeabschnitt (27) aufweist;
wobei ein Fixierabschnitt (21), der den Bedienabschnitt an einem Endoskopbedienabschnitt (15) eines Endoskops oder in der Nähe davon lösbar befestigt, an dem Bedienabschnitt vorgesehen ist.

3. Ballonkatheter nach Anspruch 1,
wobei der Fluidfreigabeabschnitt (27) mehr auf der Ballonseite angeordnet ist als der Fluidregulierungsabschnitt (28).

## Revendications

1. Cathéter à ballonnet (1) comprenant :
une tige de cathéter (3) ayant une flexibilité, avec une lumière formée à l'intérieur ;
un ballonnet (5) qui est disposé sur le côté extrémité distale de la tige de cathéter (3) et en communication avec la lumière ;
une partie de régulation de fluide (28) qui fait passer au ballonnet un fluide qui est distribué à partir d'une source d'alimentation en fluide, et, simultanément à l'arrêt de la distribution à partir de la source d'alimentation en fluide, empêche un refoulement du fluide qui est distribué au ballonnet (5) vers le côté source d'alimentation en fluide ;
une partie de libération de fluide (27) qui libère le fluide qui est distribué au ballonnet (5) vers l'extérieur du ballonnet,
une partie valve (6) qui comprend une ouverture de liaison (21) reliée au côté extrémité distale de la tige de cathéter (3), une ouverture d'attache (23) reliée à la source d'alimentation en fluide qui distribue le fluide pour dilater le ballonnet (5), et une ouverture de libération (25) reliée à l'atmosphère,
**caractérisé par le fait que**
la partie de libération de fluide (27) est disposée dans la partie valve (6) en tant que partie obturateur qui sélectionne l'une d'une première conduite (30) qui fait communiquer l'ouverture de liaison (21) avec l'ouverture d'attache (23) et d'une seconde conduite (31) qui se ramifie à partir de la première conduite (30) et fait communiquer l'ouverture de liaison (21) avec l'ouverture de libération (25), et
la partie de régulation de fluide (28) est disposée dans une position entre une position dans laquelle la partie de libération de fluide (27) est disposée et l'ouverture d'attache (23) au niveau de la première conduite (30).

2. Cathéter à ballonnet selon la revendication 1,
comportant en outre une partie d'actionnement qui a la partie de régulation de fluide (28) et la partie de libération de fluide (27) ;
une partie de fixation (21), qui monte de manière détachable la partie d'actionnement sur une partie d'actionnement d'endoscope (15) d'un endoscope ou au voisinage de celle-ci, étant située au niveau de la partie d'actionnement.

3. Cathéter à ballonnet selon la revendication 1,
dans lequel la partie de libération de fluide (27) est disposée davantage sur le côté ballonnet que la partie de régulation de fluide (28).
